# EUROPEAN PATENT SPECIFICATION

(11) **EP 0 610 447 B1**
(45) Date of publication and mention of the grant of the patent: **16.12.1998**
(21) Application number: 93908763.1
(22) Date of filing: 14.10.1992
(51) Int. Cl.: A61K 39/395

(54) **CDw52 - SPECIFIC ANTIBODY FOR TREATMENT OF T-CELL MEDIATED INFLAMMATION OF THE JOINTS**
VERWENDUNG EINES CDw52-SPEZIFISCHEN ANTIKÖRPERS ZUR BEHANDLUNG VON DURCH T-ZELLEN VERMITTELTEN GELENKENTZÜNDUNGEN
UTILISATION D'UN ANTICORPS DIRIGE CONTRE LE CDw52 POUR LE TRAITEMENT D'UNE INFLAMMATION DES ARTICULATIONS INDUITE PAR LES LYMPHOCYTES T

(30) Priority: 15.10.1991 US 775598; 24.07.1992 GB 9215749
(43) Date of publication of application: 17.08.1994
(73) Proprietor: BTG INTERNATIONAL LIMITED, London EC4M 7SB (GB)
(72) Inventor: WALDMANN, Herman, The University of Cambridge, Cambridge CB2 1QP (GB); HALE, Geoffrey, The University of Cambridge, Cambridge CB2 1QP (GB)
(74) Representative: Dolan, Anthony Patrick
(86) International application number: GB9201884
(87) International publication number: WO9307899

(56) References cited:
- EP-A- 0 328 404
- WO-A-91/10722
- WO-A-92/07084
- CLINICAL RESEARCH vol. 38, no. 2, April 1990, THOROFARE NJ, USA page 268A P. MATHIESON ET AL. 'Monoclonal antibody therapy in systemic vasculitis.'
- THE LANCET vol. 340, no. 8822, 26 September 1992, LONDON, GB pages 748 - 752 J. ISAACS ET AL. 'Humanised monoclonal antibody therapy for rheumatoid arthritis.'
- JOURNAL OF RHEUMATOLOGY vol. 18, no. 11, November 1991, TORONTO, CANADA pages 1737 - 1738 V. KYLE ET AL. 'Humanized monoclonal antibody treatment in rheumatoid arthritis.'
- J.Neuroimmunology, vol.22, p.1-9, (1989)
- New England J.Med., vol.323, p.250-254, (1990)

## Description

The present invention relates to the manufacture of a medicament for the treatment of certain auto-immune conditions in a human or animal subject.

The cells and molecules in an animal body do not normally stimulate an adaptive immune response in that body as a result of a number of mechanisms which ensure a state referred to as "self-tolerance". However, in certain circumstances these mechanisms may fail to prevent the stimulation of such an immune response and this condition is referred to as "auto-immunity". A number of diseases in humans and animals are now thought to result from auto-immunity.

One such condition is rheumatoid arthritis, a common, progressive and crippling disease, which is associated with the production by an animal of antibodies against its own IgG. The inflammation of and consequent damage to the joints associated with the disease are probably mediated via immune complexes. Diseases in which inflammation of and damage to the joints arise from auto-immunity are referred to generically herein as "T-cell mediated inflammation of the joint synovium".

The CDw52 antigen (a 23kDa glycoprotein also referred to as CAMPATH-1) is strongly expressed on the surface of all human lymphocytes and most monocytes but is absent from other blood cells including stem cells. A number of antibodies have been developed directed against CDw52 which is an unusually good target for complement-mediated attack. Antibodies against CDw52 bind to all lymphocytes and monocytes but lyse only lymphocytes (T and B) in vivo. Antigens similar to CDw52 are expressed in other mammalian species.

Antibodies which have been developed against CDw52 and which have been used in therapy include the following:
CAMPATH-1M is a rat IgM monoclonal antibody which as been used extensively in vitro for purging bone marrow harvests in order to deplete the T cell population prior to bone marrow transplantation. Marked reductions in the incidence and severity of graft-versus-host disease has been seen with this therapy.
CAMPATH-1G is a rat IgG2b class-switch variant of an IgG2a antibody recognising the CDw52 antigen which has been used in vivo to achieve immunosuppression in more than 100 patients undergoing organ and bone marrow transplantation, management of organ rejection and treatment of haematologic malignancies with a high level of success. However, the rapid development of an anti-rat immunoglobulin response, including the possibility of anaphylaxis, is likely to limit the use of rat monoclonal antibodies against the CDw52 antigen in humans in vivo.
CAMPATH-1H is a genetically manipulated IgG antibody obtained by grafting the complementarity determining regions from CAMPATH-1G into human framework regions. The resulting "humanized" antibody is highly effective in vitro being equivalent to the rat monoclonal antibody at complement lysis and two to four times better in cell-mediated lysis of human lymphocytes. No limiting antiglobulin response is anticipated with this humanized antibody.

Expression of CAMPATH-1H was achieved initially in rat myeloma cells by placing DNA encoding the engineered antibody chains in genomic context under control of the immunoglobulin promoter/enhancer. CAMPATH-1H has recently been expressed to high levels in Chinese hamster ovary (CHO) cells.

Mathieson et al, New England Journal of Medicine, 323(4), 250-254 (1990) discloses the treatment of a patient suffering from systemic vasculitis with CAMPATH-1H. Systemic vasculitis is a different and distinct condition from T-cell mediated inflammation of the joint synovium.

It has now surprisingly been found that antibodies against the CDw52 antigen are effective in the treatment of T-cell mediated inflammation of the joint synovium.

The present invention provides the use of an antibody recognising the CDw52 antigen for the manufacture of a medicament for the treatment of a human or animal subject suffering from T-cell mediated inflammation of the joint synovium.

The medicament manufactured according to the invention is applicable to the treatment of any disease involving T-cell mediated inflammation of the joint synovium. The most common such disease is rheumatoid arthritis which is a major cause of suffering and disability in the developed world. Other diseases involving T-cell mediated inflammation of the joint synovium include variants of rheumatoid arthritis such as atypical Still's disease. The medicament will find most applicability in the treatment of humans but it may also find application in veterinary medicine where animals with disease involving T-cell mediated inflammation of the joint synovium may also be treated with an appropriate antibody.

For the treatment of humans it is preferred to use an anti-CDw52 antibody which does not carry with it the risk of the development of an immune reaction against the antibody itself. Accordingly, whilst it is possible to use mouse or rat mono-clonal antibodies it is preferred to use antibodies which have been produced by recombinant DNA technology and which have been engineered to reduce the risk of causing an immune reaction. Thus it is possible to use a chimeric antibody in which the constant domains of a mouse or rat anti-CDw52 antibody have been replaced by the constant domains of a human antibody. However, it is preferred to use a humanized or CDR-grafted antibody for the treatment of humans, i.e. an antibody in which the complementarity determining regions from a mouse or rat antibody are combined with framework regions and constant domains from one or more human antibodies.

Most preferably, the antibody is the CDR-grafted antibody CAMPATH-1H (see Riechmann et al, Nature, 322, 323-327 (1988)). As noted above, the antibody CAMPATH-1H can be produced in rat myeloma cells as originally described or it can be produced in any other expression system, particularly an expression system suitable for the production of a correctly folded, glycosylated, mammalian protein. High yields of CAMPATH-1H have been obtained by expression in a genetically manipulated CHO cell line (Page and Sydenham, Biotechnology, 9, 64-68 (1991)).

The dosages of anti-CDw52 antibody, preferably CAMPATH-1H, to be administered to a patient suffering from a disease involving T-cell mediated inflammation of the joint synovium will vary with the condition being treated and the recipient of the treatment. The dose will generally be in the range 1 to about 100mg for an adult patient, usually administered daily for a period between 1 and 30 days. The preferred daily dose is 1 to 10mg per day although in some instances larger doses of up to 40mg per day may be used.

The anti-CDw52 antibody, particularly CAMPATH-1H will generally be administered to the patient in the form of a pharmaceutical formulation. Such formulations preferably include, in addition to the antibody, a physiologically acceptable carrier or diluent, possibly in admixture with one or more other agents such as other antibodies or drugs, such as an antibiotic. Suitable carriers include, but are not limited to, physiological saline, phosphate buffered saline, phosphate buffered saline glucose and buffered saline. Alternatively the antibody may be lyophilised (freeze dried) and reconstituted for use when needed by the addition of an aqueous buffered solution as described above. Routes of administration are routinely parenteral, including intravenous, intramuscular, subcutaneous and intraperitoneal injection or delivery.

The anti-CDw52 antibody may be administered in combination with or sequentially with other drugs, in particular other drugs conventionally used in the treatment of T-cell mediated inflammation of the joint synovium, such as immunosuppressive and/or anti-inflammatory agents. Typically, the immunosuppressive agents will include cyclosporin A or a purine analogue, e.g. methotrexate, 6-mercaptopurine, or the like. Suitable anti-inflammatory agents include steroids, for example hydrocortisones, such as prednisone, etc., or non-steroidal anti-inflammatory drugs, many of which are known to those skilled in the art.

Sequential administration of the antibody with another drug may be appropriate in some cases. For example, the anti-CDw52 antibody may re-establish the effectiveness of steroids in patients where steroid therapy has ceased to be effective so that treatment with the antibody may conveniently be followed by steroid therapy.

The anti-CDw52 antibody can also be administered in conjunction with another antibody having an immunosuppressive and/or tolerance inducing effect, for example anti-CD2, anti-CD3, anti-CD4 or anti-CD18 antibodies. One preferred treatment protocol according to the invention involves administration of an anti-CDw52 antibody such as Campath 1H together with or followed by administration of an anti-CD4 antibody. The administration of the antibodies may be supplemented by simultaneous or subsequent administration of a chemical therapeutic agent such as a steroid.

The invention is illustrated by the following case histories of patients treated with the CDR-grafted anti-CDw52 antibody CAMPATH-1H.

The anti-CDw52 antibody used in Case No. 1 was produced in rat Y-0 myeloma cells. The anti-CDw52 antibody used in the remaining cases was produced by expression in a recombinant CHO cell line (Page and Sydenham, Biotechnology, 9, 64-68 (1991)). In all cases the antibodies were extracted from the cell culture medium and subsequently purified.

It is to be understood that these case histories are not intended to be in any way limiting on the scope of the invention.

### CASE NO. 1

The patient was a female born in 1940 who was a typical case of a patient with severe aggressive rheumatoid arthritis who had failed to respond or had reacted adversely to several disease modifying anti-rheumatic drugs.

Immediately prior to commencement of treatment with CAMPATH-1H, the patient had restricted joint mobility and had shown a poor response to increasing doses of penicillamine which had been discontinued. Treatment with CAMPATH-1H commenced September 1988 and the patient was given 12 doses of 2mg CAMPATH-1H intravenously in 500ml normal saline over a 14 day period. This brought about a "remarkable" subjective improvement in the condition of the patient. There was also an objective improvement based on a number of conventional clinical criteria and in the sense that she was able to undertake her own self-care and also such activities as walking and knitting. The patient also received 500mg Naprosyn twice daily.

The treatment with CAMPATH-1H brought about a relatively long lasting lymphopenia. By about 20 weeks after treatment the patient's lymphocyte count had returned to above 1.0 which was matched by a reactivation of her disease. Treatment commenced with pyritinol (a second-line anti-rheumatic agent undergoing trials) and this treatment was continued for 12 months. Treatment then commenced with cyclosporin 125mg daily and 25 months after the commencement of treatment with CAMPATH-1H the patient continues to do reasonably well on cyclosporin.

### CASE NO. 2

The patient was a female born in 1971 who has been diagnosed as suffering from a particularly severe case of atypical Still's disease (a variant of rheumatoid arthritis occurring in younger patients).

At the age of 15 (in 1986) she developed fever, a brief episode of nausea and vomiting and abdominal pain and then a painful red right ankle. She was admitted to hospital and a small amount of pus was extracted from the ankle joint. At the same time she was found to have a symmetrical synovitis, right hypochondrial pain, pleuritic pain and a rash. These have persisted intermittently ever since, the polyarthropathy being dominant and immobilising.

Over the period 1988 to 1990 various investigations showed anaemia (normochronic, normocytic), thrombocytosis, hyperglobulinaemia, abnormal liver function (both alkaline phosphatase and transferases), fluctuating creatinine clearances (possibly related to NSAID therapy), hypoalbuminaemia (without proteinuria), white cell scan (uptake in joints), respiratory function tests, ECG (inverted T waves), positive ANCA. ANA, DNAB, Rh factor, complement, bone scans have always been normal as were renal, liver and skin biopsies. A synovial biopsy in June 1990 showed a predominantly T cell infiltrate and peripheral subsets showed these to be mostly T also (T 80%, B 11%).

Therapies during the above period included steroids, penicillamine, azathioprine, cyclophosphamide, methotrexate, plasma exchange, high dose intravenous gamma-globulin and cyclosporin A. Partial control was achieved with steroids, plasma exchange and cyclosporin A but remissions proved to be short lived. The progression of her disease and her lack of response to conventional and aggressive therapy resulted in her being an inpatient in hospital for long periods during 1990.

The patient commenced treatment with CAMPATH-1H in December 1990 and she received 6 daily doses of 2mg each of CAMPATH-1H followed by 6 doses of 10mg each over a 7 day period. The antibody was administered intravenously in saline. Towards the end of the treatment period a definite symptomatic improvement was noted and by the end of the treatment the patient's joints were less inflamed and she had a lower C-reactive protein value and thermographic index as well as lowered erythrocyte sedimentation rate. Shortly after the end of treatment she developed a skin abscess on her left thigh at the site of subcutaneous injection which was treated with antibiotics. Two and a half weeks after the end of treatment she changed from total parenteral nutrition to oral feeds. Four weeks after the end of treatment her condition was remarkably improved and she was discharged from hospital and allowed to return home. Steroid therapy was gradually reduced from the level prior to treatment with the antibody (30mg/day) so that five months after antibody therapy, the patient was receiving only 6mg/day prednisone and even this was subsequently withdrawn.

Approximately 9 months after the initial treatment, she suffered a relapse with multiple joint involvement. After initial testing for sensitivity with a low dose, she was given a further course of treatment of CAMPATH-1H at a dose of 8mg/day for 5 days and then 10mg/day for 20 days. The therapeutic response was good initially but the remission was relatively short lived and three months later she required further treatment.

CAMPATH-1H was given at 12mg/day for five days followed by 20mg/day of a rat derived human anti-CD4 antibody (Clone YNB46.1.8; IgG_{2b}, kappa light chain serotype; see Galfre et al., Nature, 277, 131-133 (1979)). There was no sustained therapeutic effect and investigation showed the presence of an anti-idiotypic response to CAMPATH-1H. Plasma exchange was carried out in an attempt to lower the level of the anti-idiotypic antibody and the patient was treated again with the combination of CAMPATH-1H and the anti-CD4 antibody, this time with good effect. The patient was also treated with steroid therapy (prednisone 10mg/day) and four months later was in stable remission.

### CASES NOS. 3.1 to 3.8

In a small open study, eight patients (3.1 to 3.8) with rheumatoid arthritis were treated with CAMPATH-1H. In seven out of eight case, a short course of treatment elicited favourable therapeutic responses. In only one patient was there any sign of sensitisation, and the resultant serological response was directed against a component of fetal calf serum (used in the manufacture of the antibody) and not against the humanised antibody itself.

Table 1 summarises the characteristics of the eight patients. All fulfilled the American Rheumatism Association criteria for RA and had active disease, as defined by 3 out of 4 of the following criteria: Ritchie articular index > 10, early morning stiffness > 45 minutes, ESR > 30 mm/hour, joint score > 10. Seven were seropositive. Their disease had proved unresponsive to two or more second-line agents, and these had been stopped at least four weeks before entry into the trial. Patients were permitted to continue with non-steroidal anti-inflammatory drugs (NSAID) and an existing dose of prednisolone (up to 20mg daily). There were otherwise healthy, and had normal renal and hepatic function.

Patients were admitted to hospital for antibody therapy. Therapeutic grade CAMPATH-1H was produced in Chinese hamster ovary (CHO) cells grown in hollow-fibre continuous culture system ('Acucyst-Junior', Endotronics Inc) and was purified on Protein A. It was formulated in phosphate-buffered saline and following sterility and endotoxin checks the antibody was stored at -30°C prior to administration.

The antibody was administered by intravenous infusion over a period of 2-4 hours, during which vital signs were recorded every 15 minutes. A course of therapy lasted for 10 days, and consisted of 4mg of antibody for 5 days followed by 8mg of antibody for 5 days. Between daily infusions, patients were fully mobile but did not receive physiotherapy.

Ritchie articular index and joint score were assessed immediately before treatment, and daily during treatment, by a trained metrologist. Duration of morning stiffness, patient's global assessment (comprising the sum of 4 scores representing night pain, rest pain, general well-being and functional ability, each measured on a visual analogue scale), joint thermography, ESR, CRP and FBC with differential WCC were also recorded. A similar assessment was performed weekly after therapy for one month, and then monthly. Patients were judged to have relapsed if a further second-line agent, or an increase in prednisolone dose, was administered in an attempt to control symptoms.

Patient data were analysed using the Mann-Whitney Wilcoxon Rank test, comparing values after treatment with those on day 0. Patients were excluded from further analysis if additional prednisolone or a further second-line agent was administered (i.e. if they were judged to have relapsed). This was necessary to avoid the introduction of biase by these additional therapies.

The first infusion of CAMPATH-1H resulted in a rapid fall in total lymphocyte count in all patients. Lymphopenia was evident as early as one hour after the start of the first infusion, and at this time all patients developed systemic symptoms of fever (up to 40°C), rigors, nausea and, in one case, hypotension. These symptoms lasted for 2-3 hours. Lymphocyte counts remained suppressed for several months after therapy.

In seven patients there was an impressive initial response to therapy as evidenced by clinical reduction in joint swelling and improvement in thermography. In addition, there were statistically significant improvements in Ritchie articular index and joint score lasting until day 125. Table 2 shows the pooled data for all patients and Table 1 also includes the duration of remission for individual responders, which was variable. Thus one patient (3.7) required additional therapy one month after antibody therapy, whereas another (3.5) continues to demonstrate benefit six months after treatment with sustained improvements in Ritchie index (1), morning stiffness (0 minutes), joint score (13) and global assessment (92). There was no improvement in the ESR or CRP with therapy, and no correlation between clinical relapse and lymphocyte count.

Apart from the first dose response and similar but milder symptoms following the second dose, therapy was well tolerated. The first two patients treated developed culture-negative mouth ulceration but this was not seen in subsequent patients given prophylaxis with a mixture of amphotericin and anti-bacterial mouthwashes. Mild Herpes simplex ulceration developed in patient 3.4 but responded to topical therapy with acyclovir. No adverse effects were noted on renal or hepatic function.

A weak "antiglobulin response" was detected in patient 3.5 at the end of therapy. Subsequent analysis showed that this could be adsorbed non-specifically by fetal calf serum suggesting a serological response to a component of this, rather than to CAMPATH-1H itself. Furthermore, it did not inhibit binding of CAMPATH-1H to transfectants expressing CDw52.

This is the first demonstration of an antibody treatment of rheumatoid arthritis without a significant antiglobulin response complicating the first course of therapy. The serological response measured in patient 3.5 was shown to be directed at a component of the antibody preparation other than CAMPATH-1H itself.

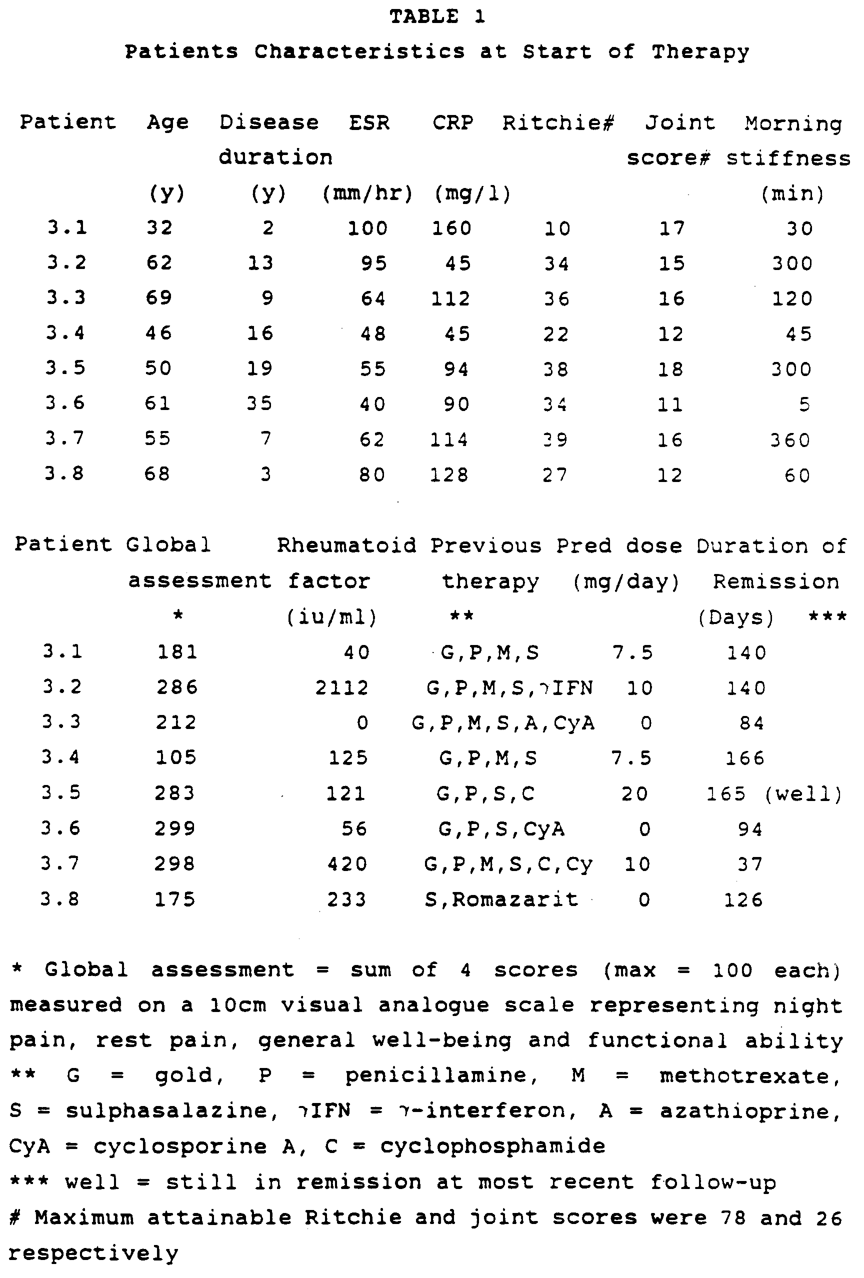

**TABLE 2**

| **Summary of Clinical Outcome. (Values are the median and range for each index at each time point).** | | | |
|---|---|---|---|
| Day# | 0(8) | 10(8) | 28(8) |
| Ritchie | 34(10-39) | 10.5(0-29)* | 9.5(1-32)* |
| Joint score | 16(12-18) | 7(1-14)* | 8.5(2-14)* |
| Morning stiffness (min) | 90(5-360) | 30(0-240) | 10(0-300) |
| Global score | 247(61-299) | 86(4-318) | 74(21-308) |
| ESR (mm/hr) | 58(40-100) | 76(8-120) | 61(20-120) |
| CRP (mg/l) | 103(45-160) | 79(23-120) | 63(23-108) |

| Day# | 56(7) | 90(7) | 125(5) |
|---|---|---|---|
| Ritchie | 9.0(0-25)* | 11.0(0-34)* | 13(2-26)* |
| Joint score | 7(0-13)* | 12(6-14)* | 12(3-14)* |
| Morning stiffness (min) | 30(0-120) | 60(0-120) | 30(0-120) |
| Global score | 59(10-368) | 126(4-395) | 136(8-271) |
| ESR (mm/hr) | 66(16-104) | 60(12-105) | 84(26-110) |
| CRP (mg/l) | 70(24-150) | 81(54-212) | 136(74-236) |

| | | | |
|---|---|---|---|
| * = significant at p<0.05 by Mann-Whitney Wilcoxon Rank test. | | | |
| # number in brackets refers to patients remaining in study at each time point. | | | |

## Claims

1. Use of an antibody recognising the CDw52 antigen for the manufacture of a medicament for the treatment of T-cell mediated inflammation of the joint synovium.

2. Use according to Claim 1, wherein said antibody is a humanised antibody.

3. Use according to Claim 2, wherein said antibody is CAMPATH-1H.

4. Use according to any of Claims 1 to 3, for the manufacture of a medicament for the treatment of rheumatoid arthritis.

5. Use according to any of Claims 1 to 4, wherein in said treatment the antibody is administered in an amount of from 1 to about 100mg per day for a period between 1 and 30 days.

6. Use according to Claim 5, wherein said amount is 1 to 10mg per day.

7. Use according to any of Claims 1 to 6, wherein said medicament is adapted to be administered intravenously.

8. Use according to any of Claims 1 to 7, wherein the medicament is for use in the treatment of T-cell mediated inflammation of the joint synovium by a method which includes simultaneous or sequential administration of another immunosuppressive antibody.

9. Use according to Claim 8, wherein said other immunosuppressive antibody is an anti-CD4 antibody.

10. Use according to any of Claims 1 to 8, wherein the medicament is for use in the treatment of T-cell mediated inflammation of the joint synovium by a method which includes simultaneous or sequential administration of a steroid.

## Patentansprüche

1. Verwendung eines Antikörpers, der das CDw52-Antigen erkennt, für die Herstellung eines Arzneimittels für die Behandlung von T-Zell-vermittelten Entzündungen des Gelenksynovialis.

2. Verwendung nach Anspruch 1, wobei der Antikörper ein humanisierter Antikörper ist.

3. Verwendung nach Anspruch 2, wobei der Antikörper CAMPATH-1H ist.

4. Verwendung nach einem der Ansprüche 1 bis 3 für die Herstellung eines Arzneimittels für die Behandlung von rheumatoider Arthritis.

5. Verwendung nach einem der Ansprüche 1 bis 4, wobei der Antikörper bei der Behandlung in einer Menge von 1 bis ungefähr 100 mg pro Tag für eine Zeitdauer zwischen 1 und 30 Tagen verabreicht wird.

6. Verwendung nach Anspruch 5, wobei die Menge 1 bis 10 mg pro Tag beträgt.

7. Verwendung nach einem der Ansprüche 1 bis 6, wobei das Arzneimittel für eine intravenöse Verabreichung angepaßt ist.

8. Verwendung nach einem der Ansprüche 1 bis 7, wobei das Arzneimittel für eine Verwendung bei der Behandlung von T-Zellvermittelten Entzündungen des Gelenksynovialis durch ein Verfahren, das eine gleichzeitige oder nacheinander erfolgende Verabreichung eines anderen immunsuppressiven Antikörpers umfaßt, bestimmt ist.

9. Verwendung nach Anspruch 8, wobei der andere immunsuppressive Antikörper ein anti-CD4-Antikörper ist.

10. Verwendung nach einem der Ansprüche 1 bis 8, wobei das Arzneimittel für eine Verwendung bei der Behandlung von T-Zellvermittelten Entzündungen des Gelenksynovialis durch ein Verfahren, das eine gleichzeitige oder nacheinander erfolgende Verabreichung eines Steroids umfaßt, bestimmt ist.

## Revendications

1. Utilisation d'un anticorps reconnaissant l'antigène CDw52 pour la préparation d'un médicament destiné au traitement de l'inflammation synoviale articulaire induite par les lymphocytes T.

2. Utilisation selon la revendication 1, dans laquelle ledit anticorps est un anticorps humanisé.

3. Utilisation selon la revendication 2, dans laquelle ledit anticorps est CAMPATH-1H.

4. Utilisation selon l'une quelconque des revendications 1 à 3, pour la préparation d'un médicament destiné au traitement de la polyarthrite rhumatoïde.

5. Utilisation selon l'une quelconque des revendications 1 à 4, dans laquelle, dans ledit traitement, l'anticorps est administré en une quantité de 1 à environ 100 mg par jour pendant une prériode comprise entre 1 et 30 jours.

6. Utilisation selon la revendication 5, dans laquelle ladite quantité est de 1 à 10 mg par jour.

7. Utilisation selon l'une quelconque des revendications 1 à 6, dans laquelle ledit médicament est sous forme appropriée pour une administration par voie intraveineuse.

8. Utilisation selon l'une quelconque des revendications 1 à 7, dans laquelle le médicament est destiné à être utilisé dans le traitement de l'inflammation synoviale articulaire induite par les lymphocytes T selon une méthode qui comprend l'administration simultanée ou séquentielle d'un autre anticorps immunosuppresseur.

9. Utilisation selon la revendication 8, dans laquelle ledit autre anticorps immunosuppresseur est un anticorps anti-CD4.

10. Utilisation selon l'une quelconque des revendications 1 à 8, dans laquelle le médicament est destiné à être utilisé dans le traitement de l'inflammation synoviale articulaire induite par les lymphocytes T selon une méthode qui comprend l'administration simultanée ou séquentielle d'un stéroïde.
